Europäisches Patentamt

European Patent Office

Office européen des brevets

(11) Veröffentlichungsnummer: **0 280 780**
**A1**

## EUROPÄISCHE PATENTANMELDUNG

(21) Anmeldenummer: 87118300.0

(22) Anmeldetag: **10.12.87**

(51) Int. Cl.⁴: **C07D 493/04** , C07D 307/20 ,
//(C07D493/04,307:00,307:00)

(30) Priorität: 04.02.87 DE 3703257

(43) Veröffentlichungstag der Anmeldung:
**07.09.88 Patentblatt 88/36**

(84) Benannte Vertragsstaaten:
**BE DE FR GB IT NL**

(71) Anmelder: **HÜLS AKTIENGESELLSCHAFT**
**Patentabteilung / PB 15 - Postfach 13 20**
**D-4370 Marl 1(DE)**

(72) Erfinder: **Lüders, Harald, Dr.**
**Langeoogstrasse 81**
**D-4350 Recklinghausen(DE)**
Erfinder: **Ratajczak, Hans-Josef, Dr.**
**Leunaer Strasse 13**
**D-4370 Marl(DE)**
Erfinder: **Wienhöfer, Ekkehard, Dr.**
**Griesheimer Strasse 12**
**D-4370 Marl(DE)**

(54) **Gezielt eingestellte Polyolgemische auf Basis von Sorbit, Verfahren zu ihrer Herstellung und ihre Verwendung zur Herstellung von Sorbitanestern.**

(57) Die bekannten Verfahren zur Herstellung von Polyolgemischen auf Basis von Sorbit mit bestimmten vorgegebenen Produkteigenschaften sind entweder ungenau oder technisch überaus aufwendig.

Die Verfolgung des Reaktionsverlaufes der Anhydrisierung an Hand des optischen Drehwertes mit Hilfe einer zuvor aufgenommenen Eichkurve ermöglicht es auf einfache Weise, Polyolgemische bestimmter Produktqualität herzustellen.

Die Polyolgemische werden zur Herstellung von Sorbitanestern mit Emulgatoreigenschaften benötigt.

EP 0 280 780 A1

## Gezielt eingestellte Polyolgemische auf Basis von Sorbit, Verfahren zu ihrer Herstellung und ihre Verwendung zur Herstellung von Sorbitanestern

Emulgatoren auf Basis von Sorbitanestern, die ganz bestimmte hydrophile und hydrophobe Eigenschaften aufweisen und vollkommen auf nachwachsender Rohstoffbasis synthetisiert werden können, finden in der Praxis großes Interesse. Es ist bekannt, daß das Eigenschaftsbild dieser Ester von der Zusammensetzung des zugrunde liegenden Polyolgemisches (Sorbit, Monoanhydrosorbit = Sorbitan, Dianhydrosorbit = Isosorbid, etc.) maßgeblich beeinflußt wird. Aus diesem Grunde ist man an Verfahren interessiert, die es gestatten, gezielt einstellbare Polyolgemische auf Basis von Sorbit herzustellen.

Es sind diverse Verfahren bekannt, um ausgehend von D-Sorbit die Anhydroformen herzustellen (siehe z. B. R. Barker, J. Org. Chem. 35, 461 (1970), J. Feldmann et al., EP-OS 0 052 295 und DE-OS 30 14 626, Soltzberg et al., J. Am. Chem. Soc. 68, 919, 927, 930 (1946) und S. Ropuszinski et al., Przem. Chem. 48, 665 (1969). Allen diesen Verfahren ist gemeinsam, daß in Gegenwart eines sauren Katalysators unter erhöhter Temperatur intramolekular Wasser abgespalten wird und stets Gemische von Anhydroformen erhalten werden. Das durch Abspaltung von Wasser erhaltene Polyolgemisch besteht nach K. Bock et al. (Acta Chem. Scand. B35, 441 - 449 (1981) beispielsweise aus

41,9 % D-Sorbit
49,0 % 1,4-Anhydro-D-sorbit
2,4 % 3,6-Anhydro-D-sorbit
3,7 % 2,5-Anhydro-L-idit
1,0 % 2,5-Anhydro-D-mannit
2,1 % 1,4 : 3,6-Dianhydro-D-sorbit

Im weiteren Verlauf der Reaktion nimmt die Konzentration an Dianhydrosorbit zu, während die des Sorbits abnimmt.

Es ist bisher weder gelungen, die Abspaltung von Wasser auf der Stufe des Monoanhydrosorbits aufzuhalten, noch die Reaktion soweit zu führen, daß ausschließlich Dianhydrosorbit gebildet wird.

Definierte Polyolgemische lassen sich im Prinzip durch Mischen der Komponenten herstellen. Diese Vorgehensweise setzt jedoch die Reinigung und Isolierung der Komponenten voraus. Da aber stets nur Gemische anfallen und eine Trennung - schwierig ist, ist diese Vorgehensweise überaus umständlich und aufwendig.

Eine Möglichkeit, derartige Gemische gezielt herzustellen, wird in der DE-OS 30 41 626 vorgeschlagen. Danach soll die Reaktionszeit der Anhydrisierung so gewählt werden, daß eine bestimmte Hydroxylzahl erreicht wird. Auch das Verfahren von G. J. Stockburger in der US-PS 42 97 290 sieht vor, Sorbit in Gegenwart von Toluolsulfonsäure solange zu erhitzen, bis sich im Reaktionsgemisch eine OH-Zahl von 1 195 eingestellt hat, und anschließend in Gegenwart von Natriumhydroxid die Veresterung durchzuführen. Die Bestimmung der OH-Zahl ist bekanntlich ein experimentell und zeitlich aufwendiges Verfahren. Es eignet sich nicht dazu, um unmittelbar den Reaktionsablauf zu verfolgen. Vielmehr ist es lediglich möglich, in Vorversuchen den Zeitpunkt zu ermitteln, zu dem eine bestimmte OH-Zahl voraussichtlich erreicht sein wird. Eine gezielte Abstoppung der Reaktion ist auf diese Weise nicht möglich. Eine Reihe von Faktoren wie z. B. Temperatur, Druck, Rührgeschwindigkeit, Konzentrationsverhältnisse, Stickstoffperlgeschwindigkeit, relative Oberfläche, Chargengröße, Wassergehalt und Qualität der Ausgangsstoffe beeinflussen in erheblicher Weise die Reaktionsgeschwindigkeit. Das Verfahren ist daher nur unter streng identischen Bedingungen reproduzierbar. Für die Praxis, in der sich solche Parameter ständig geringfügig ändern, sind diese Verfahren daher nicht geeignet. Daher wird bereits in der DE-OS 30 41 626 darauf hingewiesen, daß für jeden Einzelfall die optimale Reaktionszeit erst ermittelt werden muß (vgl. Spalte 6, Zeile 59 - 65).

Es hat Bemühungen gegeben, den Reaktionsverlauf durch zeitliche Verfolgung von Reaktionsparametern direkt zu bestimmen. Die polnischen Autoren E. Soltys et al., Tulszcze, Srodki Piorace, Kosmet, 13, 48 (1969); CA 71, 124 831 (1969) beschreiben eine solche Methode. Danach wird die Viskosität als Maß für den Reaktionsverlauf benutzt. Es ist jedoch bekannt, daß die Viskosität in erheblichem Maße von der Konzen tration des Lösemittels, hier des Wassers, abhängt. Da die Konzentration des Wassers aber gerade von den Faktoren abhängt, die auch die Reaktionsgeschwindigkeit beeinflussen, ist diese Methode zur Verfolgung der Reaktion nicht geeignet.

Flèche und Huchette verfolgen die Kinetik der Anhydrisierungsreaktion mit Hilfe der Hochdruck-Flüssigchromatographie. Dieses Verfahren ist nur diskontinuierlich durchführbar. Es liefert zwar recht zuverlässige Werte, ist aber technisch aufwendig und erfordert einen erheblichen Zeitbedarf. Für die Verfolgung der Reaktion im technischen Maßstab - scheidet es daher ebenfalls aus (vgl. Starch/Stärke 38, 26 - 30 (1986).

Ziel der vorliegenden Erfindung war es, ein Verfahren zu entwickeln, das es gestattet, die Anhydrisierung von Sorbit direkt und unmittelbar zu verfolgen, um gezielt eingestellte Polyolgemische herstellen zu können. Das Verfahren sollte einerseits ohne größeren Aufwand durchführbar sein, andererseits aber hinreichend zuverlässige Werte liefern.

Es wurde jetzt gefunden, daß der optische Drehwert in besonderer Weise zur Verfolgung des Reaktionsverlaufs geeignet ist. Dies ist überraschend, da die Anhydrisierung von Sorbit eine komplexe Reaktion ist und eine Vielzahl von Reaktionszwischen-und -endprodukten mit jeweils unterschiedlichen physikalischen Eigenschaften beobachtet werden.

Gegenstand der vorliegenden Anmeldung ist somit ein Verfahren zur Herstellung von gezielt einstellbaren Polyolgemischen, die bei der Umsetzung von D-oder L-Sorbit in Gegenwart eines sauren Katalysators bei erhöhter Temperatur anfallen. Es ist also beispielsweise möglich, ein Polyolgemisch herzustellen, das einen bestimmten vorgegebenen Gehalt an Sorbit, des Ausgangsproduktes der Anhydrisierung, aufweist. Ebenso ist es möglich, Polyolgemische mit einem bestimmten, vorgebenen Gehalt an Mono-oder Dianhydrosorbit herzustellen. Auch Gemische mit einem bestimmten Verhältnis von 2 Anhydrisierungsprodukten, wie z. B. des Verhältnisses von Mono-zu Diahydrosorbit, sind einstellbar. Schließ lich kann man nach dem vorliegenden Verfahren auch Polyolgemische mit einer bestimmten, vorgegebenen OH-Zahl herstellen. Dieses Verfahren zeichnet sich dadurch aus, daß der Reaktionsverlauf durch Messung des optischen Drehwertes verfolgt wird und die Reaktion bei Erreichen des Drehwertes abgebrochen wird, der der gewünschten Produkteigenschaft des Polyolgemisches entspricht. Die Messung des Drehwertes kann auch kontinuierlich durchgeführt werden. Bevorzugt setzt man Katalysatoren ein, die im Reaktionsgemisch löslich sind. Die erhaltenen Polyolgemische werden vorzugsweise mit Fettsäuren zu den entsprechenden Sorbitanestern umgesetzt. Von besonderem technischen Interesse sind die Gemische, die 5 bis 25 % Dianhydrosorbit enthalten. Von Interesse sind aber auch die Gemische, die einen besonders hohen Gehalt einer Komponente aufweisen und deren Isolierung aus diesem Gemisch möglich ist.

Das Verfahren weist folgende Vorteile auf:

1. Mit Hilfe einer zuvor aufgenommenen Eichkurve kann das gewünschte Polyolgemisch zuverlässig eingestellt werden.

2. Der optische Drehwert ist ohne größeren apparativen Aufwand meßbar.

3. Die Methode liefert unmittelbar und praktisch simultan einen Meßwert, der in direktem Zusammenhang mit dem gewünschten Polyolgemisch und dessen Eigenschaften steht. Es ist lediglich erforderlich, zuvor eine Eichkurve aufzunehmen.

4. Die Messung des optischen Drehwertes beeinflußt den Reaktionsverlauf nicht.

5. Der optische Drehwert ist unabhängig von den handelsüblichen Sorbitformen, die zur Herstellung von Anhydrosorbitgemischen verwendet werden.

Im folgenden soll das vorliegende Verfahren näher beschrieben werden.

Als Ausgangsstoffe eignen sich D-Sorbit und L-Sorbit. Es ist für den Fachmann ersichtlich, daß das Ausgangsprodukt möglichst nur eines der beiden Isomeren enthalten sollte. Geringe Anteile des jeweils anderen Isomeren können dann akzeptiert werden, wenn man geringere Anforderungen an die Genauigkeit der Messung stellt. Gemische mit gleichen Anteilen der beiden Isomeren sind als Ausgangsprodukt für das vorliegende Verfahren naturgemäß ungeeignet. Die meisten handelsüblichen Produkte, die in kristalliner Form, als Pulver oder als Sirup vorliegen, enthalten ausschließlich D-Sorbit. Auch die durchgeführten Versuche beziehen sich auf die D-Form. Geht man jedoch von der L-Form aus, so gelten ganz analoge Überlegungen.

Das vorliegende Meßverfahren ist im Prinzip bei allen Anhydrisierungen von Sorbit anwendbar. Im folgenden sollen übliche Reaktionsbedingungen der Anhydrisierung beschrieben werden.

Geeignete Anhydrisierungskatalysatoren sind starke Mineralsäuren oder starke organische Säuren sowie saure Ionenaustauscher, wie sie aus dem Stand der Technik bekannt sind. Bevorzugt sind Katalysatoren, die sich in der Reaktionsmischung homogen lösen, insbesondere Schwefelsäure oder p-Toluolsulfonsäure, in einer Menge von 0,01 - 1,0 %. Die Säure wird der erhitzten Sorbitschmelze in verdünnter wäßriger Form zugefügt.

Außerdem kann dem Ansatz eine geringe Menge eines Reduktionsmittels, bevorzugt 0,01 - 0,1 % Natrumhypophosphithydrat, beigefügt werden.

Die Reaktion wird vorzugsweise in einer Inertgasatmosphäre durchgeführt. Am einfachsten ist es, einen leichten Stickstoffstrom durch das Reaktionsgemisch zu leiten.

Zu Beginn der Reaktion wird die Mischung aus Sorbit, Katalysator und gegebenenfalls Reduktionsmittel auf 120 bis 160 °C erhitzt. Dabei destilliert das freiwerdende Reaktionswasser ab.

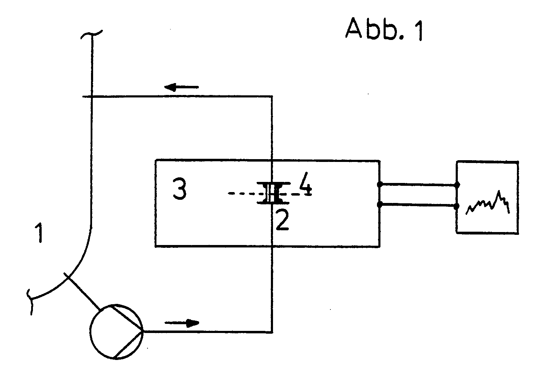

Es empfiehlt sich, die Drehwertmessung kontinuierlich durchzuführen. Die Reaktionsschmelze (1) oder zumindest ein Teil davon wird durch eine Meßzelle (2) gepumpt, die sich in einem Präzisionspolarimeter (3) befindet. Die Meßzelle, die die Form einer Küvette hat, ist so konstruiert, daß der Meßlichtstrahl (4) eine Schicht des Produktgemisches definierter Dicke (z. B. 5 mm) zwischen planparallelen Glasplättchen durchläuft (vgl. Abb. 1). Die Dicke der Küvette ist so zu bemessen, daß einerseits eine ausreichend großer Meßwert erzeugt wird und andererseits der Lichtstrahl durch Absorption nur unwesentlich geschwächt wird.

Küvette und Zuleitungsrohr sollte thermostatisiert werden, da der Drehwert in erheblichem Maße temperaturabhängig ist.

Die Messung kann jedoch auch diskontinuierlich vorgenommen werden, indem beispielsweise dem Reaktionsgemisch in kurzer zeitlicher Folge Proben entnommen und dann gemessen werden. Die Messung kann sowohl in der Schmelze, als auch in Lösung vorgenommen werden. Dem Fachmann ist bekannt, daß nur Meßwerte, die im gleichen Medium gemessen werden, miteinander vergleichbar sind. Sobald der dem gewünschten Gemisch entsprechende optische Drehwert erreicht ist, neutralisiert man das Reaktionsgemisch durch Zugabe einer äquivalenten Menge Natriumhydroxid oder einer anderen Base. Das Produkt kann anschließend durch einen Ionenaustauscher eluiert und auf diese Weise von geringen Mengen anwesender Salze gereinigt werden.

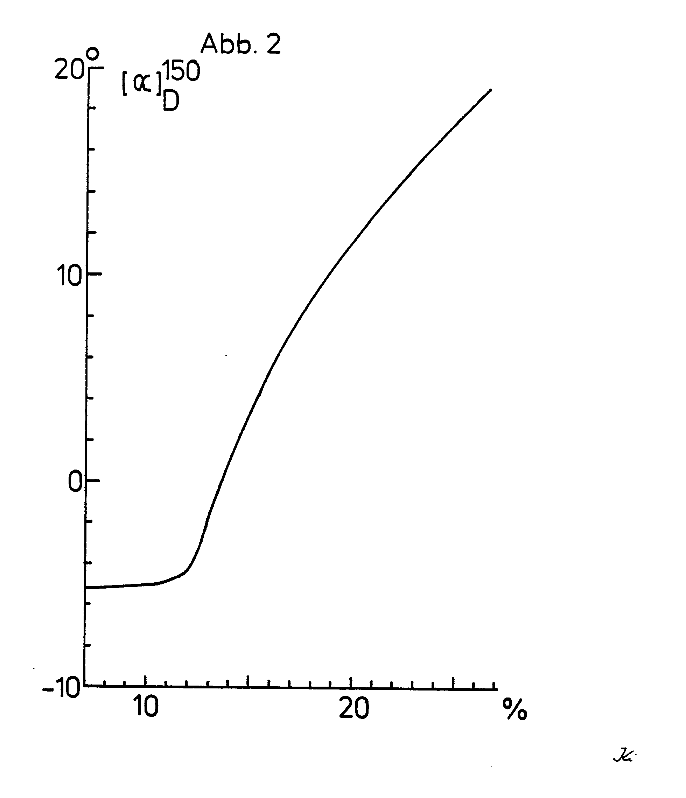

Die Abbildung 2 zeigt eine Eichkurve wie sie für den Zusammenhang zwischen Dianhydrosorbit-Anteil im Gemisch und optischem Drehwert $[Alpha]\,{}^{150}_{D}$ während der Reaktion ermittelt wurde.

Die Anhydrosorbitgemische werden insbesondere zur Herstellung von Sorbitanestern verwendet. Zur Einstellung der gewünschten Tensideigenschaften werden vorwiegend Anhydrosorbitgemische benötigt, die 5 - 25 % Dianhydrosorbit enthalten.

Die vorzugsweise rohen Gemische werden mit Fettsäuren in Gegenwart von Alkalibasen unter Erhitzen auf etwa 200 °C und kräftigem Rühren am Wasserabscheider solange erhitzt, bis die Veresterung abgeschlossen ist. Dies ist beispielsweise daran zu erkennen, daß eine Säurezahl von 2 mg KOH/g erreicht ist. Die gewünschte Farbqualität kann in bekannter Weise durch Bleichen mit Wasserstoffperoxid bei 90 °C eingestellt werden.

Beispiel 1

12,3 kg 70 %iger wäßriger Sorbitsirup und 3,3 g Natriumhypophosphithydrat werden unter Rühren und Durchleiten von Stickstoff auf 140 °C erhitzt und dabei 3,6 kg Wasser abdestilliert. Sodann werden 31,5 ml 6 N Schwefelsäure zugetropft und die Temperatur bei 135 °C gehalten. Der Drehwert der Schmelze wird kontinuierlich gemessen. Sobald bei ansteigender Drehwerttendenz der Wert $[Alpha]\,{}^{150}_{D}$ = -5,08° (in Substanz) erreicht wird, werden 31,5 ml 6 N Natronlauge zudosiert und das Produkt ausgefahren. Das Produkt weist bei einem Sorbitumsatz von 85 %, einen Dianhydrosorbitgehalt von 10 % und einen Drehwert von $[Alpha]$-

${}^{20}_{D}$ = -9,1° (Wasser) auf. Das Produkt wird zur Entsalzung in 25 l Wasser aufgenommen und durch je eine 1 000 ml-Packung stark basisches und stark saures Ionenaustauscherharz eluiert und schließlich im Wasserstrahlvakuum eingedampft.

Beispiel 2

Es wird analog Beispiel 1 verfahren. Die 6 N Natronlauge wird bei einem Drehwert $[Alpha]\,{}^{150}_{D}$ = -1,73 (in Substanz) zudosiert. Das Produkt weist bei einem Sorbitumsatz von > 97 % einen Dianhydrosorbitgehalt von 13 % und einen Drehwert von $[Alpha]\,{}^{20}_{D}$ = -6,3° (Wasser) auf.

Beispiel 3

Es wird analog Beispiel 1 verfahren. Die 6 N Natronlauge wird bei einem Drehwert von $[Alpha]\,{}^{150}_{D}$ = + 5,33° (in Substanz) zudosiert. Das Produkt weist bei einem Sorbitumsatz von > 97° einen Dianhydrosorbitgehalt von 16 % und einen Drehwert von $[Alpha]\,{}^{20}_{D}$ = + 1,97 (Wasser) auf.

Beispiel 4

Es wird analog Beispiel 1 verfahren. Die 6 N Natronlauge wird bei einem Drehwert von $[Alpha]\,{}^{150}_{D}$ = + 14,3° (in Substanz) zudosiert. Das Produkt weist bei einem Sorbitumsatz von > 97° einen Dianhydrosorbitgehalt von 26 % und einen Drehwert von $[Alpha]\,{}^{20}_{D}$ = + 14,3° auf.

**Ansprüche**

1. Verfahren zur Herstellung von gezielt einstellbaren Polyolgemischen, die bei der Anhydrisierung von D-oder L-Sorbit in Gegenwart eines sauren Katalysators bei erhöhter Temperatur anfallen, dadurch gekennzeichnet, daß man den Reaktionsverlauf durch Messung des optischen Drehwertes mit Hilfe einer zuvor aufgenommenen Eichkurve verfolgt und die Reaktion bei Erreichen des Drehwertes abbricht, der der gewünschten Produkteigenschaft des Polyolgemisches entspricht.

2. Verfahren nach Anspruch 1, dadurch gekennzeichnet, daß man folgende Produkteigenschaften des Polyolgemisches mit dem optischen Drehwert anhand einer Eichkurve korreliert:

    1. Sorbit-Gehalt

    2. Monoanhydrosorbit-Gehalt

3. Dianhydosorbit-Gehalt

4. Verhältnis von zwei Anhydrisierungsprodukten untereinander

5. OH-Zahl des Polyolgemisches

3. Verfahren nach den Ansprüchen 1 und 2, dadurch gekennzeichnet,

daß man die Messung kontinuierlich durchgeführt.

4. Verfahren nach den Ansprüchen 1 bis 3, dadurch gekennzeichnet,

daß man bei der Umsetzung von Sorbit als saure Katalysatoren solche einsetzt, die im Reaktionsgemisch homogen löslich sind.

5. Gezielt eingestellte Polyolgemische auf Basis von Sorbit, erhalten nach den Verfahren der Ansprüche 1 bis 4.

6. Verwendung der Polyolgemische gemäß Anspruch 5 zur Herstellung von Estern der Fettsäuren mit Anhydrosorbiten.

Abb. 1

Abb. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 058 246  (CPC INTERNATIONAL)<br>* Insgesamt *<br>--- | 1 | C 07 D 493/04<br>C 07 D 307/20 //<br>(C 07 D 493/04 |
| A | CHEMICAL ABSTRACTS, Band 80, Nr. 11, 18. März 1974, Seite 247, Zusammenfassung Nr. 58485e, Columbus, Ohio, US; J. DOKLADALOVA et al.: "Optical rotation assay for determination of sorbitol in the presence of mannitol and sugars", & J. ASS. OFF. ANAL. CHEM. 1973, 56(6), 1382-7<br>----- | 1 | C 07 D 307:00<br>C 07 D 307:00 ) |

**RECHERCHIERTE SACHGEBIETE (Int. Cl.4)**

C 07 D 493/00
C 07 D 307/00

**Der vorliegende Recherchenbericht wurde für alle Patentansprüche erstellt**

| Recherchenort | Abschlußdatum der Recherche | Prüfer |
|---|---|---|
| DEN HAAG | 11-05-1988 | ALLARD M.S. |